(19) Europäisches Patentamt
European Patent Office
Office européen des brevets

(11) Veröffentlichungsnummer : **0 173 202**
**B1**

# (12) EUROPÄISCHE PATENTSCHRIFT

(45) Veröffentlichungstag der Patentschrift : **28.03.90**

(51) Int. Cl.[5] : **C 07 C209/10**, C 07 C211/52

(21) Anmeldenummer : **85110345.7**

(22) Anmeldetag : **19.08.85**

## (54) Verfahren zur Herstellung von Chlor-o-nitroanilinen.

(30) Priorität : **30.08.84 DE 3431827**

(43) Veröffentlichungstag der Anmeldung : **05.03.86 Patentblatt 86/10**

(45) Bekanntmachung des Hinweises auf die Patenterteilung : **28.03.90 Patentblatt 90/13**

(84) Benannte Vertragsstaaten : **BE CH DE FR GB IT LI NL**

(56) Entgegenhaltungen :
**EP-A- 0 057 861**
**US-A- 2 305 573**
**US-A- 3 002 998**

(73) Patentinhaber : **HOECHST AKTIENGESELLSCHAFT**
**Postfach 80 03 20**
**D-6230 Frankfurt am Main 80 (DE)**

(72) Erfinder : **Warning, Klaus, Dr.**
**Kreuzheck 6**
**D-6239 Eppstein/Taunus (DE)**
Erfinder : **Folz, Georg, Dr.**
**Rauenthaler Weg 32**
**D-6000 Frankfurt am Main 71 (DE)**

**EP 0 173 202 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Verfahren zur Herstellung von Chlor-o-nitroanilinen in hoher Reinheit und Ausbeute ohne zusätzliche Reinigungsoperationen durch Umsetzung von Chlornitrobenzolen, beispielsweise von 2,4-Dichlornitrobenzol, mit wasserfreiem Ammoniak.

Chlornitroaniline sind bekanntlich wertvolle Vor- und Zwischenprodukte zur Herstellung von Farbstoffen, Pharmazeutika und Pflanzenschutzmitteln.

Es ist bekannt, beispielsweise 5-Chlor-2-nitroanilin durch Umsetzung von 2,4-Dichlornitrobenzol in ethanolischer Lösung mit Ammoniak herzustellen (A. 182 (1876) 94, 109 ; Recueil 72, 44 ff. (1953)). Diese Arbeitsweise ist jedoch mit so erheblichen Mängeln, vor allem wegen der unbegrenzten Mischbarkeit des Ethanols mit Wasser und mit dem stets im Überschuß vorhandenen Ammoniak, verbunden, daß eine technische Nutzung dieses Verfahrens nicht in Betracht kommt. Eine analoge Verfahrensdurchführung mit einer Lösung von Ammoniak in anderen wasserlöslichen niedermolekularen Alkanolen oder Glykolethern, wie beispielsweise Methanol, Isopropanol, tert. Butanol oder Diethylenglykoldimethylether, die bei der Aufarbeitung des angefallenen Reaktionsgemisches mit wasser zu mischen sind und sich dabei ebenfalls unbegrenzt mit Wasser mischen, kommt aus den genannten Gründen technisch auch nicht in Frage.

Bei der Herstellung von 5-Chlor-2-nitroanilin durch Ammonolyse in wäßrigen Systemen (Recueil 72, 44 ff. (1953)) entstehen daneben immer auch, und zwar weitgehend unabhängig von den Reaktionsbedingungen, erhebliche Mengen an 3-Chlor-4-nitroanilin und 5-Amino-2-nitroanilin (Diamin), wobei bei sehr schonender Reaktionsführung auch noch nicht umgesetztes Ausgangsprodukt vorhanden sein kann.

Die Reinigung derartiger Gemische ist technisch nur unter beträchtlichem Aufwand und unter Minderung der Ausbeute durchzuführen, verbunden auch mit erheblichen Umweltproblemen. Außerdem besitzen im Vergleich zu reinem 5-Chlor-2-nitroanilin derart erhaltene Reaktionsmischungen stark herabgesetzte thermische Stabilitäten, so daß sich bei Durchführung solcher Verfahren im technischen Maßstab sicherheitstechnische Probleme ergeben.

Nach der europäischen Patentanmeldung 0 057 861 (US-Patentschrift 4 421 694) erhält man 5-Chlor-2-nitroanilin durch Umsetzung von 2,4-Dichlornitrobenzol in chlorierten aromatischen Kohlenwasserstoffen mit Ammoniak in Ausbeuten bis zu ca. 95 % und Reinheiten bis zu 96 %. Neben der im Vergleich zu den genannten bekannten Verfahren zwar gesteigerten, jedoch noch nicht zufriedenstellenden Selektivität bestehen auch Bedenken hinsichtlich der Anwendung von chlorierten Kohlenwasserstoffen aus gewerbehygienischer Sicht. So liegen beispielsweise die Werte der maximalen Arbeitsplatzkonzentration für Chlorbenzol und die Dichlorbenzole mit 50 ppm bemerkenswert niedrig. Allgemein besteht die Tendenz, den Einsatz von chlorierten Kohlenwasserstoffen aus Gründen geringerer Gesundheitsrisiken auf ein unabdingbares Minimum zu beschränken.

Es wurde nun überraschenderweise gefunden, daß man Chlor-o-nitroaniline der allgemeinen Formel (1)

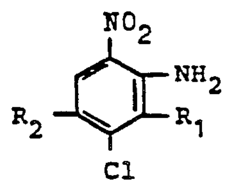

(1)

in welcher $R_1$ und $R_2$ Wasserstoffatome oder Chloratome bedeuten, in nahezu quantitativer Ausbeute und fast 100-%iger Reinheit herstellen kann, indem man Chlornitrobenzole der allgemeinen Formel (2)

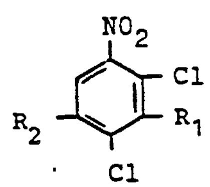

(2)

in welcher $R_1$ und $R_2$ die vorstehend genannten Bedeutungen haben, mit wasserfreiem Ammoniak in Gegenwart aromatischer Kohlenwasserstoffe der allgemeinen Formel (3)

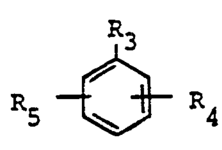

(3)

in welcher $R_3$ eine Alkyl$_{C1-C4}$ Gruppe, wie beispielsweise die Methyl, Ethyl, n-Butyl-, iso-Butyl-, tert. Butyl-, Propyl- oder iso-Propylgruppe, darstellt, und $R_4$ und $R_5$ Wasserstoffatome oder Alkyl$_{C1-C4}$ Gruppen bedeuten, bei Temperaturen von etwa 120 bis 200 °C, vorzugsweise 150 bis 180 °C, umsetzt.

An Chlornitrobenzolen der genannten allgemeinen Formel (2) seien beispielsweise das 2,3,4- und 2,4,5-Trichlornitrobenzol, das 2,3,4,5-Tetrachlornitrobenzol und insbesondere das 2,4-Dichlornitrobenzol genannt.

Das jeweils zur Nitro-Gruppe p-ständige und bekanntlich ebenfalls aktivierte Chlor-Atom greift unter den Bedingungen des erfindungsgemäßen Verfahrens in die Reaktion praktisch nicht ein.

An aromatischen Kohlenwasserstoffen der genannten allgemeinen Formel (3) seien beispielsweise genannt Toluol, Ethylbenzol, Xylole und Mesitylen, wobei Toluol und Xylole bevorzugt eingesetzt werden.

Es können auch Mischungen von Kohlenwasserstoffen der genannten allgemeinen Formel (3) angewandt werden.

Die verfahrengemäß bevorzugt zur Anwendung gelangenden aromatischen Kohlenwasserstoffe der allgemeinen Formel (3) sind allgemein verfügbar, kostengünstig und aus gewerbehygienischer Sicht weniger problematisch als die chlorierten aromatischen Kohlenwasserstoffe.

Sowohl das einzusetzende Ammoniak als auch die angewandten aromatischen Kohlenwasserstoffe müssen weitestgehend wasserfrei sein, da mit zunehmender Anwesenheit von Wasser der selektive Austausch des zur Nitrogruppe o-ständigen aktivierten Chloratoms durch die Aminogruppe in den Ausgangsprodukten der genannten allgemeinen Formel (2) zurückgeht.

Die Wassermengen, die beim Aufarbeiten des anfallenden Reaktionsgemisches in den aromatischen Kohlenwasserstoffen gelöst bleiben, sind jedoch für das erfindungsgemäße Verfahren unschädlich.

Die Menge der aromatischen Kohlenwasserstoffe kann in weiten Grenzen variiert werden. Vorteilhaft wählt man die Mengenverhältnisse so, daß nach dem Abkühlen des angefallenen Reaktionsgemisches noch gut rühr- und fließfähige Suspensionen erhalten werden.

Maßgebende Parameter für die Geschwindigkeit und Vollständigkeit des Umsatzes sind die Reaktionstemperatur und die Menge des eingesetzten Ammoniaks. Obwohl im Prinzip die stöchiometrische Menge von 2 Mol $NH_3$ pro Mol Chlornitrobenzol der Formel (2) ausreicht, ist es zweckmäßig, eine über dieses Verhältnis hinausgehende Menge zu verwenden. Das Molverhältnis von Ausgangsprodukt zu Ammoniak kann dabei von etwa 1 : 3 bis 1 : 12 variieren, wobei man zweckmäßigerweise ein Molverhältnis von 1 : 6 bis 1 : 8 wählt. Nicht verbrauchtes Ammoniak kann nach üblichen Methoden zurückgewonnen und wieder eingesetzt werden.

Die Reaktionstemperatur kann sich innerhalb eines weiten Bereichs bewegen. Zweckmäßigerweise werden Temperaturen zwischen etwa 120 und 200 °C gewählt; bevorzugt wird die Umsetzung bei Temperaturen von 150-180 °C durchgeführt. Zusammen mit einem entsprechend hohen Überschuß an Ammoniak ergeben sich dann Reaktionszeiten von ca. 3-6 Stunden. Die sehr hohe Selektivität wird weder von der Höhe der Temperatur noch von der Menge des eingesetzten Ammoniaks signifikant beeinflußt. Die Kombination dieser Parameter bestimmt lediglich die Vollständigkeit des Umsatzes bzw. die erforderliche Reaktionsdauer.

Das erfindungsgemäße Verfahren kann im einzelnen so ausgeführt werden, daß man das Ausgangsprodukt (Chlornitrobenzol der allgemeinen Formel (2)) zusammen mit dem aromatischen Kohlenwasserstoff der Formel (3) in einem Autoklav vorlegt und das wasserfreie Ammoniak auf einmal oder in Anteilen zudosiert.

Die Isolierung des Reaktionsproduktes erfolgt nach üblichen Methoden. So kann beispielsweise nach dem Entspannen des Autoklavs — das überschüssige Ammoniak kann dabei zurückgewonnen und einem anderen Ansatz zugeführt werden — und Abkühlen der angefallenen Reaktionsmischung das Produkt aus der erhaltenen Kristallsuspension abfiltriert und das mitausgefallene Ammoniumchlorid mit Wasser ausgewaschen werden. Das Filtrat, das überwiegend aus dem aromatischen Kohlenwasserstoff besteht, ist ohne weitere Maßnahmen wieder einsetzbar. Besonders vorteilhaft gestaltet sich die Aufarbeitung durch Entfernen des aromatischen Kohlenwasserstoffs durch Wasserdampf-Destillation und Abfiltrieren des Produkts aus der wäßrigen Suspension. Auch hier kann der Kohlenwasserstoff nach Abtrennung vom Wasser direkt wieder für nachfolgende Chargen verwendet werden. Die im Kohlenwasserstoff gelösten Spuren von Wasser stören dabei im Sinne des erfindungsgemäßen Verfahrens nicht.

Besonders vorteilhaft gegenüber den zum Stand der Technik zählenden Verfahren ist beim erfindungsgemäßen Verfahren die hohe Selektivität, mit der die Reaktion abläuft. Bei beinahe quantitativem Umsatz findet Diaminierung praktisch nicht statt, so daß aufwendige Reinigungsoperationen unterbleiben können. Die Reaktion ist über weite Temperatur- und Konzentrationsbereiche unkritisch und erfordert nur allgemein zugängliche und kostengünstige aromatische Kohlenwasserstoffe als Reaktionsmedium. Durch die Vermeidung der Bildung von Diaminonitrobenzolen wird eine stark erhöhte thermische Stabilität erzielt und die Neigung zu unkontrollierten Zersetzungsreaktionen unterdrückt, die bei den nach den bekannten Verfahren erhaltenen Gemischen gegeben ist.

In den nachfolgenden Beispielen wird das erfindungsgemäße Verfahren verdeutlicht, ohne darauf beschränkt zu werden. Prozentangaben beziehen sich auf das Gewicht.

## Beispiel 1

In einem Stahlautoklav werden 192 g (1 Mol) 2,4-Dichlornitrobenzol und 400 g Xylol vorgelegt. Nach dem Verschließen des Autoklavs drückt man 136 g (8 Mol) Ammoniak-Gas auf und erhitzt 5 Stunden auf 170 °C, wobei sich ein Druck von ca. 70 bar einstellt. Anschließend wird abgekühlt, entspannt und das

Xylol mit Wasserdampf abgetrieben. Aus der wäßrigen Kristallsuspension wird das Produkt durch Filtration erhalten und mit Wasser chloridfrei gewaschen.

Man erhält 171 g 5-Chlor-2-nitroanilin vom Schmelzpunkt 124,2 °C, was einer Ausbeute von 99 % der Theorie entspricht. Nach gaschromatographischer Analyse liegt der Gehalt an 5-Chlor-2-nitroanilin bei 98,8 % (Diamin-Gehalt < 0,2 %).

Beispiel 2

Erhitzt man statt 5 Stunden bei 170 °C 3 Stunden bei 180 °C und verfährt im übrigen wie in Beispiel 1 beschrieben, so erhält man das 5-Chlor-2-nitroanilin in gleicher Ausbeute und Reinheit wie gemäß Beispiel 1.

Beispiel 3

192 g (1 Mol) 2,4-Dichlornitrobenzol werden zusammen mit 300 g Toluol in einen Stahlautoklav gegeben. Nach Aufdrücken von 102 g (6 Mol) Ammoniak wird 6 Stunden auf 170 °C erhitzt. Nach der in Beispiel 1 beschriebenen Aufarbeitung des angefallenen Reaktionsgemisches erhält man das 5-Chlor-2-nitroanilin in gleicher Ausbeute und Reinheit wie gemäß Beispiel 1.

**Patentansprüche**

1. Verfahren zur Herstellung von Chlor-o-nitroanilinen der allgemeinen Formel (1)

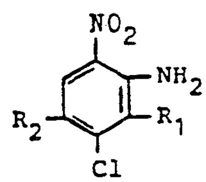

(1)

in welcher $R_1$ und $R_2$ Wasserstoffatome oder Chloratome bedeuten, durch Umsetzung von Chlornitrobenzolen der allgemeinen Formel (2)

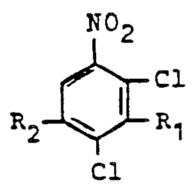

(2)

in welcher $R_1$ und $R_2$ die vorstehend genannten Bedeutungen haben, mit Ammoniak bei erhöhter Temperatur, dadurch gekennzeichnet, daß man die Umsetzung mit wasserfreiem Ammoniak in Gegenwart aromatischer Kohlenwasserstoffe der allgemeinen Formel (3)

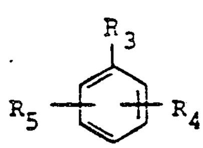

(3)

in welcher $R_3$ eine $Alkyl_{C1-C4}$ Gruppe darstellt und $R_4$ und $R_5$ Wasserstoffatome oder $Alkyl_{C1-C4}$ Gruppen bedeuten, bei Temperaturen von etwa 120 bis 200 °C vornimmt.

2. Verfahren nach Anspruch 1, dadurch gekennzeichnet, daß man die Umsetzung bei Temperaturen von 150 bis 180 °C vornimmt.

3. Verfahren nach Anspruch 1, dadurch gekennzeichnet, daß man die Chlornitrobenzole der genannten Formel (2) und das Ammoniak im Molverhältnis 1 : 3 bis 1 : 12 einsetzt.

4. Verfahren nach Anspruch 1, dadurch gekennzeichnet, daß man die Chlornitrobenzole der genannten Formel (2) und das Ammoniak im Molverhältnis 1 : 6 bis 1 : 8 einsetzt.

5. Verfahren nach Anspruch 1, dadurch gekennzeichnet, daß man die Umsetzung in Gegenwart von Toluol oder Xylolen durchführt.

## Claims

1. A process for the preparation of chloro-o-nitroanilines of the formula (1)

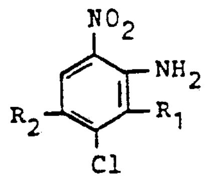

(1)

in which $R_1$ and $R_2$ are hydrogen atoms or chlorine atoms, by reaction of chloronitrobenzenes of the formula (2)

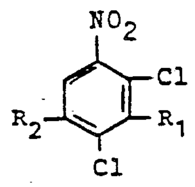

(2)

in which $R_1$ and $R_2$ have the meanings given above, with ammonia at an elevated temperature, which comprises effecting the reaction at temperatures from about 120 to 200 °C with anhydrous ammonia in the presence of aromatic hydrocarbons of the formula (3)

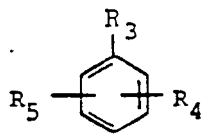

(3)

in which $R_3$ is a $C_1$-$C_4$-alkyl group and $R_4$ and $R_5$ are hydrogen atoms or $C_1$-$C_4$-alkyl groups.

2. The process as claimed in claim 1, wherein the reaction is carried out at temperatures from 150 to 180 °C.

3. The process as claimed in claim 1, wherein the chloronitrobenzenes of the said formula (2) and the ammonia are employed in a molar ratio of 1 : 3 to 1 : 12.

4. The process as claimed in claim 1, wherein the chloronitrobenzenes of the said formula (2) and the ammonia are employed in a molar ratio of 1 : 6 to 1 : 8.

5. The process as claimed in claim 1, wherein the reaction is carried out in the presence of toluene or xylenes.

## Revendications

1. Procédé pour préparer des chloro-o-nitranilines répondant à la formule générale 1 :

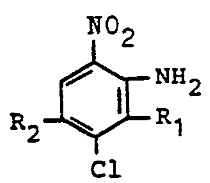

(1)

dans laquelle $R_1$ et $R_2$ représentent chacun un atome d'hydrogène ou un atome de chlore, par réaction de chloro-nitrobenzènes répondant à la formule générale

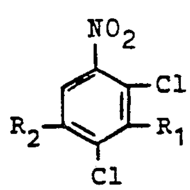

(2)

dans laquelle $R_1$ et $R_2$ ont les significations qui leur ont été données ci-dessus, avec l'ammoniac, à température élevée, procédé caractérisé en ce qu'on effectue la réaction avec de l'ammoniac anhydre, en présence d'hydrocarbures aromatiques répondant à la formule générale 3 :

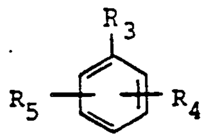

(3)

dans laquelle $R_3$ représente un radical alkyle en $C_1$-$C_4$, et $R_4$ et $R_5$ représentent chacun un atome d'hydrogène ou un radical alkyle en $C_1$-$C_4$, à des températures d'environ 120 à 200 °C.

2. Procédé selon la revendication 1 caractérisé en ce qu'on effectue la réaction à des températures de 150 à 180 °C.

3. Procédé selon la revendication 1 caractérisé en ce que les chloro-nitrobenzènes de formule générale 2 et l'ammoniac sont mis en jeu dans un rapport molaire compris entre 1 : 3 et 1 : 12.

4. Procédé selon la revendication 1 caractérisé en ce que les chloro-nitrobenzènes de formule générale 2 et l'ammoniac sont mis en jeu dans un rapport molaire compris entre 1 : 6 et 1 : 8.

5. Procédé selon la revendication 1 caractérisé en ce qu'on effectue la réaction en présence de toluène ou de xylènes.